# EUROPÄISCHE PATENTSCHRIFT

(11) **EP 1 556 016 B1**
(45) Veröffentlichungstag und Bekanntmachung des Hinweises auf die Patenterteilung: **03.09.2008**
(21) Anmeldenummer: 03809716.8
(22) Anmeldetag: 04.09.2003
(51) Int. Cl.: A61K 9/50, A61K 31/573

(54) **MEHRSCHICHTIGE WIRKSTOFFHALTIGE ARZNEIFORMEN, DIE EINEN NEUTRALEN KERN SOWIE EINEN INNEREN UND ÄUSSEREN ÜBERZUG AUS METHACRYLAT-COPOLYMEREN UND -MONOMEREN UMFASSEN**
MULTILAYER DOSAGE FORMS, WHICH CONTAIN ACTIVE SUBSTANCES AND WHICH COMPRISE A NEUTRAL CORE, AND AN INNER AND OUTER COATING CONSISTING OF METHACRYLATE COPOLYMERS AND METHACRYLATE MONOMERS
FORMES GALENIQUES MULTICOUCHE A BASE DE PRINCIPE ACTIF, COMPRENANT UN NOYAU NEUTRE ET UN REVETEMENT INTERIEUR ET EXTERIEUR A BASE DE COPOLYMERES ET DE MONOMERES METHACRYLATE

(30) Priorität: 29.10.2002 DE 10250543
(43) Veröffentlichungstag der Anmeldung: 27.07.2005
(73) Patentinhaber: Evonik Röhm GmbH, 64293 Darmstadt (DE)
(72) Erfinder: PETEREIT, Hans-Ulrich, 64291 Darmstadt (DE); RUDOLPH, Markus, 1267 Vich (CH); DRESSMAN, Jennifer, 60322 Frankfurt (DE); BECKERT, Thomas, 88447 Warthausen (DE)
(86) Internationale Anmeldenummer: PCT/EP2003/009800
(87) Internationale Veröffentlichungsnummer: WO 2004/039357

(56) Entgegenhaltungen:
- EP-A- 0 519 870
- US-A- 5 643 602

## Beschreibung

Die Erfindung betrifft eine mehrschichtige Arzneiform mit neutralem Methacrylat-Copolymer als Bindemittel für den Wirkstoff.

### Stand der Technik

Die Verwendung von sogenannten neutralen Methacrylat-Copolymeren, das heißt Methacrylat-Copolymeren, die zum überwiegenden Teil aus (mindestens 95%) (Meth)acrylat-Monomeren mit neutralen Resten, wie Methylmethacrylat oder Ethylacrylat, bestehen, als Überzugs- und Bindemittel für Arzneiformen mit verzögerter Wirkstofffreisetzung ist seit langem bekannt.

Verwendungen in Mischungen mit anionischen Dispersionen sind beschrieben z. B. in EP-A 152 038, EP-A 208 213 oder EP-A 617 972.

WO 01/68767 beschreibt eine Dispersion, geeignet zur Verwendung als Überzugs- und Bindemittel für Arzneiformen, mit einem Feststoffgehalt von 10 - 70 Gew.-% bestehend aus
a) 90 bis 99 Gew.-% eines Methacrylat-Copolymeren, das zu mindestens zu 90 Gew.-% aus (Meth)acrylat-Monomeren mit neutralen Resten besteht und eine Glastemperatur Tg von - 20 °C bis + 20 °C bestimmt nach der DSC-Methode und
b) 1 - 10 Gew.-% eines nichtionischen Emulgators mit einem HLB-Wert von 15, 2 bis 17,3.

Die Verwendung der speziellen Emulgatoren gemäß der WO 01/68767 erlaubt es unter Beibehaltung der Stabilität der Dispersion und ihrer Teilchengrößenverteilung, daraus Arzneimittelformulierungen herzustellen, bei denen eine Phasenseparation unter Ausbildung von Kristallstrukturen durch den Emulgator unterbleibt.

Die WO 01/68767 erwähnt weiterhin, daß Schichten von mehrlagigen Überzugssystemen erzeugt werden können. Beispielsweise kann ein Kern, der z.B. basische oder wasserempfindliche Wirkstoffe enthält, mit einer Isolierschicht aus einem anderen Überzugsmaterial, wie Celluloseether, Celluloseester, kationische Polymethacrylate (wie EUDRAGIT^{®} E I00, -RL 100, - RS 100, Röhm GmbH), versehen werden, bevor das erfindungsgemäße Überzugsmittel aufgetragen wird. Ebenso können anschließend weitere Überzüge, beispielsweise mit geruchs- oder geschmackskaschierender Wirkung oder mit ansprechender Farb- oder Glanzwirkung, aufgebracht werden.

Ein typisches Methacrylat-Copolymer gemäß der WO 01/68767 kann z. B. aus 25 - 35 Gew.-% Methylmethacrylat und 75 bis 65 Gew.-% Ethylacrylat aufgebaut sein. Gegebenenfalls können auch geringe Comonomer-Anteile anderer Vinylmonomere enthalten sein.

Mehrschichtige Arzneiformen sind hinlänglich bekannt. Die WO 01/68058 beschreibt z. B. die Verwendung einer mehrschichtigen Arzneiform, die im wesentlichen aufgebaut ist aus
a) einem Kern mit einem pharmazeutischen Wirkstoff
b) einem inneren Überzug aus einem Copolymeren oder einer Mischung von Copolymeren, die sich aus 85 bis 98 Gew.-% radikalisch polymerisierten C1- bis C4-Alkylestern der Acryl- oder der Methacrylsäure und 15 bis 2 Gew.-% (Meth)acrylat-Monomeren mit einer quaternären Ammoniumgruppe im Alkylrest zusammensetzen und einem
c) einem äußeren Überzug aus einem Copolymeren, das sich aus 75 bis 95 Gew.-% radikalisch polymerisierten C₁- bis C₄-Alkylestern der Acryl- oder der Methacrylsäure und 5 bis 25 Gew.-% (Meth)acrylat-Monomeren mit einer anionischen Gruppe im Alkylrest zusammensetzt
zur Herstellung einer Arzneiform, welche im Freisetzungstest nach USP zwei Stunden bei pH 1,2 und anschließendem Umpuffern auf pH 7,0 den enthaltenen Wirkstoff im Zeitraum bis 2,0 Stunden nach Testbeginn zu weniger als 5 % und zum Zeitpunkt acht Stunden nach Testbeginn zu 30 bis 80 % freisetzt.

US5643602 und EP0519870 offenbaren einen Kern, der mit zwei Polymerschichten umgeben ist. Die erste Schicht kann nach Auswahl aus Listen aus Eudragit NE 30 D (EP0519870) bzw. Eudragit NE (siehe US5643602), die zweite Schicht ebenso nach Auswahl aus Listen aus Eudragit L 30 D (siehe EP0519870) sowie Eudragit S, Eudragit L 100-55 (siehe US5643602) bestehen.

US5643602 verwendet Budesonid und Glukokortikoide in den entsprechenden pharmazeutischen Formulierungen zur Behandlung von Morbus Crohn sowie Colitis ulcerosa, EP0519870 Diclofenac Natrium in einer eigenen Wirkstoffschicht, die den Kern unmittelbar umgibt.

### Aufgabe und Lösung

Die vorliegende Erfindung geht von der WO 01/68767 aus. Die darin beschriebene mehrschichtige Arzneiform erlaubt die Einstellung variabler Freisetzungsprofile und eine präzise und unter definierten Bedingungenreproduzierbare Wirkstoffabgabe.

Ihre Herstellung ist durch den in mehreren Arbeitsgängen herzustellenden Mehrschichtenaufbau vergleichsweise aufwendig.

Nach Auslösung der äußeren Überzugsschicht ist zudem nicht völlig auszuschließen, daß es je nach eingestellten Schichtdicken, Zusammensetzungen, dem jeweiligen Wirkstoff und dessen Konzentration zu Wechselwirkungen zwischen der inneren Überzugsschicht und den aus dem Kern retardierend freigesetzten Wirkstoffmolekülen kommen kann. Dies scheint

insbesondere bei Wirkstoffen mit polaren oder ionischen Molekülgruppen der Fall zu sein, die mit den positiv geladenen quaternären Ammoniumgruppen der (Meth)acrylat-Copolymere oder deren Chlorid-Gegenionen in Wechselwirkungen treten können.

Ein weiteres Problem besteht in einer offenbar durch die Ionenstärke des umgebenden Mediums beeinflussten Wirkstofffreigabecharakteristik. Da gerade orale Arzneiformen häufig mit Wasser eingenommen und auch die Ionenstärke in Magen und Darm z. B. durch die Nahrungsaufnahme immer gewissen Schwankungen unterliegen, sind die Arzneiformen in vivo wechselnden Ionenstärken ausgesetzt. Dies kann, in vivo, zu nicht immer reproduzierbaren Wirkstofffreigabecharakteristiken führen. Wünschenswert sind deshalb Arzneiformen, deren Wirkstofffreigabecharakteristiken weitestgehend unbeeinflußt von der Ionenstärke des umgebenden Mediums bleiben.

Hinzu kommt, daß die innere Überzugsschicht in der Regel mit Hilfe von Weichmachern formuliert werden muß, um eine ausreichende Flexibiltät der Filme zu gewährleisten. Auch der Einsatz von Trennmitteln wie z. B. Talkum oder Glycerolmonostearat ist in der Regel unumgänglich, um ein Verkleben der überzogenen Einheiten bei oder nach dem Auftrag der inneren Überzugsschicht zu verhindern.

Es wurde daher als Aufgabe gesehen, eine mehrschichtige Arzneiform zu entwickeln, die ähnlich wie die der WO 01/68767 die Einstellung variabler Freisetzungsprofile und eine präzise, auch bei unterschiedlichen Ionenstärken des umgebenden Mediums reproduzierbare Wirkstoffabgabe erlaubt. Die mehrschichtige Arzneiform sollte jedoch vergleichsweise einfacher herstellbar sein. Zudem sollten mögliche Wechselwirkungen zwischen dem enthaltenen Wirkstoff und den mit dem Wirkstoff in Kontakt tretenden polymeren Überzugsmitteln oder Weichmachern gering gehalten oder vermieden werden können.

Die Aufgabe wird gelöst durch eine
mehrschichtige Arzneiform, aufgebaut aus
a) einem neutralen Kern,
b) einem inneren Überzug aus einem Methacrylat-Copolymeren
c) einem äußeren Überzug aus einem Copolymeren, das sich aus 40 bis 95 Gew.-% radikalisch polymerisierten C₁- bis C₄-Alkylestern der Acryl- oder der Methacrylsäure und 5 bis 60 Gew.-% (Meth)acrylat-Monomeren mit einer anionischen Gruppe im Alkylrest zusammensetzt
   **dadurch gekennzeichnet, daß**
der innere Überzug im wesentlichen aus einem Methacrylat-Copolymeren besteht, das mindestens zu 90 Gew.-% aus (Meth)acrylat-Monomeren mit neutralen Resten aufgebaut ist, eine Mindestfilmbildetemperatur nach DIN 53 787 von höchstens 30 °C aufweist und den pharmazeutischen Wirkstoff in gebundener Form enthält.

Gegenüber der mehrschichtige Arzneiform gemäß WO 01/68767 ist die erfindungsgemäße Arzneiform leichter herstellbar, da der Wirkstoff in einem Arbeitsschritt mit dem inneren Polymerüberzug aufgetragen werden kann. Die Verwendung eines Methacrylat-Copolymeren, das mindestens zu 90 Gew.-% aus (Meth)acrylat-Monomeren mit neutralen Resten aufgebaut ist, eine Mindestfilmbildetemperatur nach DIN 53 787 von höchstens 30 °C aufweist, ermöglicht es weitgehend oder sogar völlig auf Hilfsstoffe wir Weichmacher oder Trennmittel zu verzichten. Vorteilhafter Weise und nicht vorhersehbar wird nach Auflösung des äußeren Überzugs im Colon eine ähnlich retardierende Freisetzung des im Polymer gebundenen Wirkstoffs erhalten, wie sie bei WO 01/68767 mit einem im Kern gebundenen Wirkstoff und einem Überzug aus (Meth)acrylat-Copolymeren mit quaternären Aminogruppen möglich ist. Ein wichtiger Vorteil der erfindungsgemäßen Arzneiform besteht darin, daß die Wirkstofffreigabe bei konstantem pH-Wert in einem hypotonischen und einem isotonischen Medium nahezu nicht von der Ionenstärke beeinflußt wird.

### Ausführung der Erfindung

### Die Erfindung betrifft eine

Mehrschichtige Arzneiform, die einen enthaltenen pharmazeutischen Wirkstoff im Freisetzungstest nach USP zwei Stunden bei pH 1,2 und anschließendem Umpuffern auf einen pH von mindestens 6,8 den enthaltenen Wirkstoff im Zeitraum bis 2,0 Stunden nach Testbeginn zu weniger als 5 % und zum Zeitpunkt acht Stunden nach Testbeginn zu 30 bis mindestens 80 % freisetzt
und aufgebaut ist aus
a) einem neutralen Kern,
b) einem inneren Überzug aus einem Methacrylat-Copolymeren
c) einem äußeren Überzug aus einem Copolymeren, das sich aus 75 bis 95 Gew.-% radikalisch polymerisierten C₁- bis C₄-Alkylestern der Acryl- oder der Methacrylsäure und 5 bis 60 Gew.-% (Meth)acrylat-Monomeren mit einer anionischen Gruppe im Alkylrest zusammensetzt
   **dadurch gekennzeichnet, daß**
der innere Überzug im wesentlichen aus einem Methacrylat-Copolymeren besteht, das mindestens zu 90 Gew.-% aus (Meth)acrylat-Monomeren mit neutralen Resten aufgebaut ist, eine Mindestfilmbildetemperatur nach DIN 53 787 von höchstens 30 °C aufweist und den pharmazeutischen Wirkstoff in gebundener Form enthält.

### Kerne a)

Träger bzw. neutrale Kerne für die Überzüge sind Tabletten, Granulate, Pellets, Kristalle von regelmäßiger oder unregelmäßiger Form. Die Größe von Granulaten, Pellets oder Kristallen liegt in der Regel zwischen 0,01 und 2,5 mm, die von Tabletten zwischen 2,5 und 30,0 mm.

Die Kerne können weitere pharmazeutische Hilfsstoffe enthalten: Bindemittel, wie Lactose, Cellulose und deren Derivate, Polyvinylpyrrolidon (PVP), Feuchthaltemittel, Zerfallsförderer, Gleitmittel, Sprengmittel, Stärke und deren Derivate, Zucker Solubilisatoren oder andere enthalten.

### Innerer Überzug b)

Der inneren Überzug b) besteht im wesentlichen aus einem Methacrylat-Copolymeren, das zu mindestens zu 90 Gew.-% aus (Meth)acrylat-Monomeren mit neutralen Resten besteht und eine Mindestfilmbildetemperatur nach DIN 53 787 von höchstens 30 °C, besonders bevorzugt höchstens 25 °C aufweist, mit einem darin gebundenen pharmazeutischen Wirkstoff.

Die Schichtdicke des inneren Überzugs kann bevorzugt zwischen 10 und 300 µm betragen

### Methacrylat-Copolymer für den inneren Überzug b)

Das Methacrylat-Copolymer für den inneren Überzug b) besteht mindestens zu 90, insbesondere zu 95, bevorzugt zu 97, insbesondere zu 99, besonders bevorzugt zu 100 Gew.-% aus (Meth)acrylat-Monomeren mit neutralen Resten, insbesondere C₁- bis C₄-Alkylresten.

Geeignete Monomere sind z. B. Methylmethacrylat, Ethylmethacrylat, Butylmethacrylat, Methylacrylat, Ethylacrylat, Butylacrylat. Bevorzugt sind Methylmethacrylat, Ethylacrylat und Methylacrylat.

Die an sich neutralen Polymere können geringe Mengen Methacrylsäure oder Acrylsäure enthalten, die zwar die Wasserunlöslichkeit des Polymeren praktisch nicht verändern, jedoch die Quellung beeinflußen können und eine pHabhängige Steuerung der Permeabilität erlauben.

In geringen Anteilen, zu höchstens 10, bevorzugt höchstens 5, besonders bevorzugt höchstens 3 oder höchstens 1 Gew.-% können andere vinylisch polymerisierbare Monomere, insbesondere (Meth)acrylatmonomere mit polaren oder ionischen Resten, z. B. Methacrylsäure oder Acrylsäure, enthalten sein.

Das (Meth)acrylat-Copolymere weist eine Mindestfilmbildetemperatur nach DIN 53 787 von höchstens 30 °C aufweist

Das Methacrylat-Copolymer kann bevorzugt eine Glastemperatur Tg von - 25 °C bis + 20 °C, bevorzugt - 10 °C bis 0 °C, bestimmt nach der DSC-Methode (ISO 11357) aufweisen.

Das Methacrylat-Copolymer des inneren Überzugs kann z. B. aus 25 - 35 Gew.-% Methylmethacrylat, 75 bis 65 Gew.-% Ethylacrylat und nicht mehr als 1 Gew.-% Methacrylsäure polymerisiert sein, wobei sich die Mengenanteile zu 100 Gew.-% addieren.

Das (Meth)acrylat-Copolymer für den inneren Überzug b) kann als organische Lösung oder als Dispersion vorliegen und verarbeitet werden.

Bevorzugt setzt man das (Meth)acrylat-Copolymer für den inneren Überzug b) in Form einer Dispersion, mit einem Feststoffgehalt von 10 - 70 Gew.-% ein.

Die entsprechende Dispersion enthält besonders bevorzugt 1 bis 10, bevorzugt 2 bis 8, besonders bevorzugt 4 bis 6 Gew.-% bezogen auf den Feststoffanteil eines nichtionischen Emulgators mit einem HLB-Wert von 15, 7 bis 19,5. Geeignet ist z. B. Polyoxyethylen-100-isononylphenol (HLB ca. 19,1).

### Emulgatoren

Emulgatoren kontrollieren den Ablauf des Emulsionspolymerisationsverfahrens, im dem sie die kettenaufbauende Reaktion der emulgierten Monomere in der Wasserphase ermöglichen. Sie sind daher ein für die Herstellung notwendiger Hilfsstoff und bestimmend für die Eigenschaften der Dispersion. Sie können üblicherweise nicht ausgetauscht werden, ohne relevante Eigenschaften der Dispersion grundlegend zu verändern.

Der HLB-Wert ist ein 1950 von Griffin eingeführtes Maß der Hydrophilie bzw. Lipophilie von nichtionischen Tensiden. Er läßt sich experimentell durch die Phenol-Titrationsmethode nach Marszall bestimmen; vgl. "Parfümerie, Kosmetik", Band 60, 1979, S. 444 - 448; weitere Literaturhinweise in Römpp, Chemie-Lexikon, 8.Aufl. 1983, S.1750. Siehe weiterhin z. B. US 4 795 643 (Seth)).

Ein HLB-Wert (Hydrophile/Lipophile Balance) läßt sich nur bei nicht ionischen Emulgatoren exakt bestimmen. Bei anionischen Emulgatoren kann dieser Wert rechnerisch ermittelt werden, liegt jedoch praktisch immer über oder weit über 20.

Die HLB-Werte der Emulgatoren haben einen deutlichen Einfluß auf die Auskristallisation des Emulgators. Im Idealfall liegen diese Werte zwischen 15, 7 und 16,2. Oberhalb des beanspruchten Bereiches kristallisieren die Emulgatoren nach dem Trocknen aus. Emulgatoren mit einem HLB-Wert unterhalb des beanspruchten Bereiches können die Dispersion nicht ausreichend stabilisieren was an starker Koagulatbildung zu erkennen ist. Die HLB Werte wurden entweder der Literatur (Fiedler: Lexikon der Hilfsstoffe) entnommen bzw. nach W. C. Griffin (Sonderdruck aus Parfümerie und Kosmetik 64, 311-314, 316 (1983); Hüthig Verlag, Heidelberg / Pharmind Ind. 60 Nr.1 (1998); Dielektrizitätsthermoanalyse) berechnet.

Der Emulgator soll toxikologisch unbedenklich und daher bevorzugt nichtionisch Emulgatoren sein.

Geeignete Emulgatorklassen sind ethoxylierte Fettsäureester oder -ether, ethoxylierte Sorbitanether, ethoxylierte Alkylphenole, Glycerin- oder Zuckerester oder Wachsderivate Geeignete Emulgatoren sind zum Beispiel Polyoxyethylenglycerinmonolaurat, Polyoxyethylenglycerinmonostearat, Polyoxyethylen-20-cetylstearat Polyoxyethylen-25-cetylstearat, Polyoxyethylen(25)oxypropylenmonostearat, Polyoxyethylen-20-sorbitanmonopalmitat, Polyoxyethylen-16-tert.oktylphenol, Polyoxyethylen-20-cetylether, Polyethylenglykol(1000)monocetylether, ethoxyliertes Rizinusöl, Polyoxyethylensorbitol-Wollwachs-Derivate, Polyoxyethylen(25)propylenglykolstearat und Polyoxyethylensorbitester

Bevorzugt sind Polyoxyethylen-25-cetylstearat, Polyoxyethylen-20-sorbitanmonopalmitat, Polyoxyethylen-16-tert.oktylphenol und Polyoxyethylen-20-cetylether.

### Herstellung einer Dispersion

Eine Dispersion wird in an sich bekannter Weise durch wäßrige Emulsionspolymerisation im Batch- oder im Zulaufverfahren, halbkontinuierlich oder auch kontinuierlich gewonnen (s. dazu z.B. DE 195 03 099 A1).

Die radikalische Polymerisation der Monomeren in Gegenwart des Emulgators erfolgt mittels radikalbildender wasserlöslicher Polymerisationsinitiatoren, bei denen die Radikalbildung thermisch oder über Redoxprozesse erfolgen kann. Gegebenenfalls werden Molekulargewichts-Regler zur Einstellung der Molmassen zugesetzt. Emulsionspolymerisate werden üblicherweise in Konzentrationen zwischen 10 und 70 Gew.% hergestellt. Günstig ist ein Feststoffgehalt von 30 - 50 Gew.%. Die diskontinuierliche Herstellung erfolgt in der Regel in Rührkessel-Reaktoren.

Zur Herstellung werden bei einer einfachen Batchherstellung alle Monomeren gemäß der gewünschten Copolymerzusammensetzung zusammen mit dem Emulgator, Initiatoren, Reglern und sonstigen Hilfsmitteln zusammen mit Wasser in einem Reaktionskessel vorgelegt und darin gelöst bzw. dispergiert. Durch Aktivierung des Starters (Erhöhung der Temperatur, Zugabe des Redoxmittels) wird die polymere Kettenreaktion initiiert und durchgeführt. Hierbei bilden sich die bekannten aus Polymerketten bestehenden Latexteilchen aus.

Der Dispersion können Antischaumemulsion und Stabilisatoren zugegeben werden.

### Herstellung der erfindungsgemäßen Arzneiform

### Sprühauftrag

Die für die Umsetzung der Erfindung notwendigen Auftragsverfahren entsprechen dem Stand der Technik und sind beispielsweise in folgenden Lehrbüchern beschrieben:
Bauer, Lehmann, Osterwald, Rothgang, "Überzogene Arzneiformen" Wissenschaftliche Verlagsgesellschaft mbH Stuttgart, Kap 7, S.165 - 196
Bauer, Lehmann, Osterwald, Rothgang, "Coated Pharmaceutical Dosage Forms" CRS Press 1988, Chapter 7
J. W. McGinity (Ed.), Aqueous Coatings for Pharmaceutical Dosage Forms, Marcel Dekker Inc., 1997
K. Lehmann et al., "Practical Course in Film Coating of Pharmaceutical Dosage Forms with EUDRAGIT® ", RÖHM GmbH & Co. KG., 2001
M. Dombrow (Ed.) Microcapsules and Nanoparticles in Medicine and Pharmacy, CRS Press,1992

### Weiterverarbeitung zu oralen Arzneiformen

Zur Anwendung kommen Verfahren nach dem üblichen Stand der Technik. Details sind den gängigen Lehrbüchern zu entnehmen, z. B.:
- Voigt, R. (1984): Lehrbuch der pharmazeutischen Technologie; Verlag Chemie Weinheim - Beerfield Beach/Florida - Basel.
- Sucker, H., Fuchs, P., Speiser, P.: Pharmazeutische Technologie, Georg Thieme Verlag Stuttgart (1991), insbesondere Kapitel 15 und 16, S. 626 - 642.
- Gennaro, A.,R. (Editor), Remington's Pharmaceutical Sciences, Mack Publishing Co., Easton Pennsylvania (1985), Chapter 88, S. 1567 - 1573.
- List, P. H. (1982): Arzneiformenlehre, Wissenschaftliche Verlagsgesellschaft mbH, Stuttgart.

Von bevorzugter Bedeutung sind dabei das Verpressen zu Tabletten und das Abfüllen in Kapseln.
Für die Applikation relevante Eigenschaften, geforderte Tests und Spezifikationen sind in Arzneibüchern aufgelistet.

### Bindung des Wirkstoffs

Die Bindung des Wirkstoffs erfolgt bevorzugt durch wäßriges Sprühen einer Wirkstoff-haltigen (Meth)acrylat-Copolymer-Dispersion auf die Kerne a), z. B. Saccharose Pellets, unter Bindung des Wirkstoffs nach dem Abdampfen bzw. der Verflüchtigung des Wassers. Die Produkttemperatur während des Sprühauftrages kann dabei z. B. 20 bis 40, bevorzugt 25 bis 35 °C betragen.

Eine Variante des Verfahrens ist das sogenannte Powder-Layering-Verfahren, bei dem die (Meth)acrylat-Copolymer-Dispersion gesprüht wird und dabei der Wirkstoff in Pulverform zugegeben wird.

In der Regel kann man bei der Verarbeitung der Wirkstoffhaltigen (Meth)acrylat-Copolymer-Dispersion auf Trennmittel, wie z. B. Talkum oder auf einen Weichmacher-Zusatz verzichten.

Die Verarbeitung des Wirkstoffs kann bevorzugt durch Einrühren in Wasser unter zunächst heftiger Durchmischung, z. B. durch 5 bis 15 minütiges Mischen z. B. mit einem Hochgeschwindigkeitsmixer (Homogenisator) erfolgen. Die so erhaltene Suspension oder Lösung kann dann der (Meth)acrylat-Copolymer-Dispersion zugegeben werden. Die Mischung sollte zweckmäßigerweise und bevorzugt auch während des Sprühvorgangs kontinuierlich gerührt werden. Außerdem kann ein wasserlöslicher Wirkstoff in gelöster Form in die Polymerdispersion gegeben werden und anschließend aufgesprüht werden.

Der Wirkstoff liegt im Copolymer des inneren Überzugs b) entweder in kristalliner Form vor (solid dispersion) oder in gelöster Form (solid solution) vor.

Das Wirkstoff/Polymerverhältnis der inneren Schicht kann 20 zu 1 bis 1 zu 20, bevorzugt 1 zu 1 bis 1 zu 3 betragen.

### Pharmazeutische Wirkstoffe

Die erfindungsgemäße Arzneiform eignet sich zur Verabreichung grundsätzlich beliebiger pharmazeutischer Wirkstoffe, die vorzugsweise im Dünndarm und/oder Colon freigesetzt werden sollen, und insbesondere solcher, die mit Vorteil in retardierter Form verabreicht werden können, wie Antidiabetika, Analgetika, Antiphlogistika, Antirheumatika, Antihypotonika, Antihypertonika, Psychopharmaka, Tranquilizer, Antiemetika, Muskelrelaxantien, Glucocorticoide, Mittel zur Behandlung von Colitis ulcerosa oder Morbus Crohn, Antiallergika, Antibiotika, Antiepileptika, Antikoagulantia, Antimykotika, Antitussiva, Arteriosklerosemittel, Diuretika, Enzyme, Enzyminhibitoren, Gichtmittel, Hormone und deren Hemmstoffe, Herzglykoside, Immuntherapeutika und Zytokine, Laxantien, Lipidsenker, Migränemittel, Mineraistoffpraparate, Otologika, Parkinsonmittel, Schilddrüsentherapeutika, Spasmolytika, Thrombozytenaggregationshemmer, Vitamine, Zytostatika und Metastasenhemmer, Phytopharmaka, Chemotherapeutika und Aminosäuren.

Beispiele geeigneter Wirkstoffe sind Acarbose, Betarezeptorenblocker, Nichtsteroidale Antirheumatia, Herzglykoside, Acetylsalicylsäure, Virustatika, Aclarubicin, Acyclovir, Cisplatin, Actinomycin, alpha- und beta-Sympatomimetika, (Dmeprazol, Allopurinol, Alprostadil, Prostaglandine, Amantadin, Ambroxol, Amlodipin, Methotrexat, S-Aminosalicylsäure, Amitriptylin, Amoxicillin, Anastrozol, Atenolol, Azathioprin, Balsalazid, Beclomethason, Betahistin, Bezafibrat, Bicalutamid, Diazepam und Diazepamderivate, Budesonid, Bufexamac, Buprenorphin, Methadon, Calciumsalze, Kaliumsalze, Magnesiumsalze, Candesartan, Carbamazepin, Captopril, Cefalosporine, Cetirizin, Chenodeoxycholsäure, Ursodeoxycholsäure, Theophyllin und Theophyllinderivate, Trypsine, Cimetidin, Clarithromycin, Clavulansäure, Clindamycin, Clobutinol, Clonidin, Cotrimoxazol, Codein, Coffein, Vitamin D und Derivate von Vitamin D, Colestyramin, Cromoglicinsäure, Cumarin und Cumarinderivate, Cystein, Cytarabin, Cyclophosphamid, Ciclosporin, Cyproteron, Cytarabin, Dapiprazol, Desogestrel, Desonid, Dihydralazin, Diltiazem, Mutterkornalkaloide, Dimenhydrinat, Dimethylsulfoxid, Dimeticon, Dipyridarnoi, Domperidon und Domperidanderivate, Dopamin, Doxazosin, Doxorubizin, Doxylamin, Dapiprazol, Benzodiazepine, Diclofenac, Glykosidantibiotika, Desipramin, Econazol, ACE-Hemmer, Enalapril, Ephedrin, Epinephrin, Epoetin und Epoetinderivate, Morphinane, Calciumantagonisten, Irinotecan, Modafinil, Orlistat, Peptidantibiotika, Phenytoin, Riluzole, Risedronat, Sildenafil, Topiramat, Makrolidantibiotika, Estrogen und Estrogenderivate, Gestagen und Gestagenderivate, Testosteron und Testosteronderivate, Androgen und Androgenderivate, Ethenzamid, Etofenamat, Etofibrat, Fenofibrat, Etofyllin, Etoposid, Famciclovir, Famotidin, Felodipin, Fenofibrat, Fentanyl, Fenticonazol, Gyrase-Hemmer, Fluconazol, Fludarabin, Flunarizin, Fluorouracil, Fluoxetin, Flurbiprofen, Ibuprofen, Flutamid, Fluvastatin, Follitropin, Formoterol, Fosfomicin, Furosemid, Fusidinsäure, Gallopamil, Ganciclovir, Gemfibrozil, Gentamicin, Ginkgo, Johanniskraut, Glibenclamid, Harnstoffderivate als orale Antidiabetika, Glucagon, Glucosamin und Glucosaminderivate, Glutathion, Glycerol und Glycerolderivate, Hypothalamushormone, Goserelin, Gyrasehemmer, Guanethidin, Halofantrin, Haloperidol, Heparin und Heparinderivate, Hyaluronsäure, Hydralazin, Hydrochlorothiazid und Hydrochlorothiazidderivate, Salicylate, Hydroxyzin, Idarubicin, Ifosfamid, Imipramin, Indometacin, Indoramin, Insulin, Interferone, Jod und Jodderivate, Isoconazol, Isoprenalin, Glucitol und Glucitolclerivate, Itraconazol, Ketoconazol, Ketoprofen, Ketotifen, Lacidipin, Lansoprazol, Levodopa, Levomethadon, Schilddrüsenhormone, Liponsäure und Liponsäurederivate, Lisinopril, Lisurid, Lofepramin, Lomustin, Loperamid, Loratadin, Maprotilin, Mebendazol, Mebeverin, Meclozin, Mefenaminsäure, Mefloquin, Meloxicam, Mepindolol, Meprobamat,Meropenem, Mesalazin, Mesuximid, Metamizol, Metformin, Methotrexat, Methylphenidat, Methylprednisolon, Metixen, Metoclopramid, Metoprolol, Metronidazol, Mianserin, Miconazol, Minocyclin, Minoxidil, Misoprostol, Mitomycin, Mizolastin, Moexipril, Morphin und Morphinderivate, Nachtkerze, Nalbuphin, Naloxon, Tilidin, Naproxen, Narcotin, Natamycin, Neostigmin, Nicergolin, Nicethamid, Nifedipin, Nifluminsäure, Nimodipin, Nimorazol, Nimustin, Nisoldipin, Adrenalin und Adrenalinderivate, Norfloxacin, Novaminsulfon, Noscapin, Nystatin, Ofloxacin, Olanzapin, Olsalazin, Omeprazol, Omoconazol, Ondansetron, Oxaceprol, Oxacillin, Oxiconazol, Oxymetazolin, Pantoprazol, Paracetamol, Paroxetin, Penciclovir, orale Penicilline, Pentazocin, Pentifyllin, Pentoxifyllin, Perphenazin, Pethidin, Pflanzenextrakte, Phenazon, Pheniramin, Barbitursäurederivate, Phenylbutazon, Phenytoin, Pimozid, Pindolol, Piperazin, Piracetam, Pirenzepin, Piribedil, Piroxicam, Pramipexol, Pravastatin, Prazosin, Procain, Promazin, Propiverin, Propranolol, Propyphenazon, Prostaglandine, Protionamid, Proxyphyllin, Quetiapin, Quinapril, Quinaprilat, Ramipril, Ranitidin, Reproterol, Reserpin, Ribavirin, Rifampicin, Risperidon, Ritonavir, Ropinirol, Roxatidin, Roxithromycin, Ruscogenin, Rutosid und Rutosidderivate, Sabadilla, Salbutamol, Salmeterol, Scopolamin, Selegilin, Sertaconazol, Sertindol, Sertralion, Silikate, Simvastatin, Sitosterin, Sotalol, Spagluminsäure, Sparfloxacin, Spectinomycin, Spiramycin, Spirapril, Spironolacton, Stavudin, Streptomycin, Sucralfat, Sufentanil, Sulbactam, Sulfonamide, Sulfasalazin, Sulpirid, Sultamicillin, Sultiam, Sumatriptan, Suxamethoniumchlorid, Tacrin, Tacrolimus, Taliolol, Tamoxifen, Taurolidin, Tazaroten, Temazepam, Teniposid, Tenoxicam, Terazosin, Terbinafin, Terbutalin, Terfenadin, Terlipressin, Tertatolol, Tetracycline, Tetryzolin, Theobromin, Theophyllin, Butizin, Thiamazol, Phenothiazine, Thiotepa, Tiagabin, Tiaprid, Propionsaurederivate, Ticlopidin, Timolol, Tinidazol, Tioconazol, Tioguanin, Tioxolon, Tiropramid, Tizanidin, Tolazolin, Tolbutamid, Tolcapon, Tolnaftat, Tolperison, Topotecan, Torasemid, Anti6strogene, Tramadol, Tramazolin, Trandolapril, Tranylcypromin, Trapidil, Trazodon, Triamcinolon und Triamcinolonderivate, Triamteren, Trifluperidol, Trifluridin, Trimethoprim, Trimipramin, Tripelennamin, Triprolidin, Trifosfamid, Tromantadin, Trometamol, Tropalpin, Troxerutin, Tulobuterol, Tyramin, Tyrothricin, Urapidil, Ursodeoxycholsäure, Chenodeoxycholsäure, Valaciclovir, Valproinsäure, Vancomycin, Vecuroniumchlorid, Viagra, Venlafaxin, Verapamil, Vidarabin, Vigabatrin, Viloxazin, Vinblastin, Vincamin, Vincristin, Vindesin, Vinorelbin, Vinpocetin, Viquidil, Warfarin, Xantinolnicotinat, Xipamid, Zafirlukast, Zalcitabin, Zidovudin, Zolmitriptan, Zolpidem, Zoplicon, Zotepin und dergleichen.

Beispiele besonders bevorzugter Wirkstoffe sind Mittel zur Behandlung von Colitis ulcerosa oder Morbus Crohn wie Salicylate z.B. 5-Aminosalicylsäure, 4-Aminosalicylsäure, Olsalazin, Balsalazin, Sulfasalazin, Corticosteroide, wie Budesonid, Prednisolon, Methylprednisolon, Prednison, Dexamethason, Hydrocortison, Triamcinolon, Antiasthmatika wie Theophyllin und Salbutamol, Analgetika, wie Tramadol, Morphin, Codein, Protonenpumpen-Inhibitoren, wie Omeprazol, Virusstatika, wie Amantadin, Memantadin, Ribavirin und Acyclovir, Lipidsenker, wie Simvastatin oder Pravastatin, H2-Blocker, wie Ranitidin oder Famotidin, Antibiotika, wie Makrolide: Erythromycin, Azithromycin Clarithromycin, Roxithromycin, Tetracycline, wie Doxycyclin, Minocyclin, Tetracyclin, wie Gyrasehemmer: Ciprofloxacin, Ofloxacin, β-Lactame: wie Penicilline, z. B. Phenoxyphenylpenicillin, Cephalosporine, z. B. Cefaclor, Cefalexin, Cefadroxil, Cefixim und Inhibitoren z.B. Sulbactam, Sultamicillin, Clavulansäure, Aminoglycoside wie Gentamycin, Nitroimidazol-Derivate wie MetamizolACE-Hemmer, wie Enalapril oder Amlodipin, Immunmodulatoren wie Azathioprin, Methotrexat, Cyclosporin, Tacrolismus, Diclizimab und Infliximab, Calciumantagonisten, wie Nifedipin, Nimodipin und Nircardipin, Beta-Blocker wie Atenolol, Betaxolol, Metoprolol, Oxprenolol, Nebvolol und Propranolol, Peptide oder Hormone wie Pankreatin, ein Insulin, ein Human Growth Hormon (hGH), Corbaplatin, Intron A, Calcitonin, Cromalyn, ein Interferon, ein Calcitonin, Granulocyte Colony Stimulating factor (G-CSF), ein Interleukin, Parathyroidhormone, Glucagon, Pro-Somatostatin, ein Somatostatin, Detirelix, Cetrorelix, Vasopressin, 1-Deaminocysteine-8-D-arginine-Vasopressin, Leuprolidacetat oder ein Antigen, das aus Gräsern oder anderen Pflanzen, wie z. B. Roggen, Weizen, Gerste, Hafer, Bermuda Gras, Zinnkraut, Ahorn, Ulme, Eiche, Platane, Pappel, Zeder, Zinnkraut, Disteln gewonnen wurde, sein.

Die Wirkstoffe können gewünschtenfalls auch in Form ihrer pharmazeutisch annehmbaren Salze oder Derivate verwendet werden, und im Falle chiraler Wirkstoffe können sowohl optisch aktive Isomere als auch Racemate oder Diastereoisomerengemische eingesetzt werden. Gewünschtenfalls können die erfindungsgemässen Zusammensetzungen auch zwei oder mehrere pharmazeutische Wirkstoffe enthalten.

### Äußerer Überzug c)

Der äußere Überzug c) besteht in wesentlichen aus (Meth)acrylat-Copolymeren, welche aus 40 bis 95 Gew.-% radikalisch polymerisierten Einheiten von C₁- bis C₄-Alkylestern der Acryl- oder der Methacrylsäure und 5 bis 60 Gew.-% (Meth)acrylat-Monomeren mit einer anionischen Gruppe im Alkylrest bestehen. In der Regel addieren sich die genannten Anteile zu 100 Gew.-%. Es können jedoch zusätzlich, ohne daß dies zu einer Beeinträchtigung oder Veränderung der wesentlichen Eigenschaften führt, geringe Mengen im Bereich von 0 bis 10, z. B. 1 bis 5 Gew.-% weiterer vinylisch copolymerisierbarer Monomere, wie z. B. Methylmethacrylat, Butylmethacrylat, Butylacrylat oder Hydroxyethylmethacrylat enthalten sein.

C₁- bis C₄-Alkylestern der Acryl- oder Methacrylsäure sind insbesondere Methylmethacrylat, Ethylmethacrylat, Butylmethacrylat, Methylacrylat, Ethylacrylat und Butylacrylat.

Ein (Meth)acrylat-Monomer mit einer anionischen Gruppe im Alkylrest kann z. B. Acrylsäure, bevorzugt jedoch Methacrylsäure sein. Die Carboxylgruppen können bis zu 30 mol-%, bevorzugt bis zu 5 bis 15 mol-% teilneutralisiert sein.

Geeignet sind anionische (Meth)acrylat Copolymere aus 40 bis 60, Gew.-% Methacrylsäure und 60 bis 40 Gew.-% Methylmethacrylat oder 60 bis 40 Gew.-% Ethylacrylat (Typen EUDRAGIT® L oder EUDRAGIT® L 100-55).

Ebenso geeignet sind anionische (Meth)acrylat Copolymere aus 20 bis 40 Gew.-% Methacrylsäure und 80 bis 60 Gew.-% Methylmethacrylat (Typ EUDRAGIT® S).

Geeignet sind ebenfalls anionische (Meth)acrylat Copolymere aus 20 bis 34 Gew.-% Methacrylsäure und/oder Acrylsäure, 20 bis 69 Gew.-% Methylacrylat, 0 bis 40 Gew.-% Ethylacrylat und gegebenenfalls 0 bis 10 Gew.-% weiteren vinylisch copolymerisierbaren Monomeren, mit der Maßgabe, daß die Glastemperatur des Copolymers nach ISO 11357-2, Punkt 3.3.3, höchstens 60 °C beträgt. (Typ EUDRAGIT® mit mittlerem Gehalt an Methacrylsäure).

Das Copolymer setzt sich insbesondere zusammen aus radikalisch polymerisierten Einheiten von

20 bis 34, bevorzugt 25 bis 33, besonders bevorzugt 28 bis 32 Gew.-% Methacrylsäure oder Acrylsäure, bevorzugt ist Methacrylsäure,

20 bis 69, bevorzugt 35 bis 65, besonders bevorzugt 35 bis 55 Gew.-% Methylacrylat und gegebenenfalls

0 bis 40, bevorzugt 5 bis 35, besonders bevorzugt 15 bis 35 Gew.-% Ethylacrylat zusammen, mit der Maßgabe, daß die Glastemperatur des Copolymers (ohne Weichmacherzusatz) nach ISO 11357-2, Punkt 3.3.3, höchstens 60, bevorzugt 40 bis 60, besonders bevorzugt 45 bis 55 °C beträgt.

Besonders geeignet sind weiterhin auch z. B. (Meth)acrylat Copolymere, bestehend aus 10 bis 30 Gew.-%, Methylmethacrylat, 50 bis 70 Gew.-% Methylacrylat und 5 bis 15 Gew.-% Methacrylsäure (Typ EUDRAGIT® FS).

Die erfindungsgemäße Arzneiform mit den genannten äußeren Überzügen, insbesondere dem Typ, bestehend aus 10 bis 30 Gew.-%, Methylmethacrylat, 50 bis 70 Gew.-% Methylacrylat und 5 bis 15 Gew.-% Methacrylsäure (Typ EUDRAGIT® FS) eignet sich besonders für Arzneiformen, die den Wirkstoff im distalen Ileum oder Colon freisetzen.

Die erfindungsgemäße Arzneiform mit den genannten äußeren Überzügen, insbesondere dem Typ, bestehend aus 10 bis 30 Gew.-%, Methylmethacrylat, 50 bis 70 Gew.-% Methylacrylat und 5 bis 15 Gew.-% Methacrylsäure (Typ EUDRAGIT® FS) eignet sich besonders für Arzneiformen, die folgende Wirkstoffklassen und Wirkstoffe enthalten und zur Therapie des Morbus Crohn oder der Colitis Ulcerosa eingesetzt werden können. Zu nennen sind die Wirkstoffklassen der Aminosalicylate, der Sulfonamide oder der Glucocorticoide. Besonders bevorzugte Wirkstoffe sind 5-Aminosalicylsäure, Olsalazin, Sulfalazin, Prednison, Prednisolon oder Budesonid.

Die Arzneiform eignet sich insbesondere für immunmodulatorische Wirkstoffe aus den Stoffklassen Proteine, Peptide, Oligonukleotide mit einem vermuteten Wirkort an der Darmmucosa und speziell an den "Payer's Plaques" in der Colonmucosa.

Die erfindungsgemäße Arzneiform mit den genannten äußeren Überzügen, insbesondere der Typ, bestehend aus 10 bis 30 Gew.-%, Methylmethacrylat, 50 bis 70 Gew.-% Methylacrylat und 5 bis 15 Gew.-% Methacrylsäure (Typ EUDRAGIT® FS) eignen sich besonders für Arzneiformen, die folgende Wirkstoffklassen und Wirkstoffe enthalten. Zu nennen sind die Wirkstoffklassen Enzyme, ein Peptidhormone, immunmodulatorische Proteine, Antigene, Antikörper oder Oligonukleotide.

Besonders bevorzugte Wirkstoffe sind Pankreatin, Insulin, Human Growth Hormon (hGH), Corbaplatin, Intron A, Calcitonin, Cromalyn, Interferone, Calcitonin, Granulocyte Colony Stimulating factor (G-CSF), Interleukin, Parathyroidhormone, Glucagon, Pro-Somatostatin, Somatostatin, Detirelix, Cetrorelix, Vasopressin, 1-Deaminocysteine-8-D-arginine-Vasopressin, Leuprolidacetat oder ein Antigen, das aus Gräsern oder anderen Pflanzen, wie z. B. Roggen, Weizen, Gerste, Hafer, Bermuda Gras, Zinnkraut, Ahorn, Ulme, Eiche, Platane, Pappel, Zeder, Zinnkraut, Disteln gewonnen wurde.

Das (Meth)acrylat-Copolymer des äußeren Überzugs c) besteht bevorzugt in wesentlichen bis ausschließlich aus den Monomeren Methacrylsäure, Acrylsäure, Methylmethacrylat, Methylacrylat und/oder Ethylacrylat in den oben angegebenen Mengenanteilen. In der Regel addieren sich die genannten Anteile zu 100 Gew.-%. Es können jedoch zusätzlich, ohne daß dies zu einer Beeinträchtigung oder Veränderung der wesentlichen Eigenschaften führt, geringe Mengen im Bereich von 0 bis 10, z. B. 1 bis 5 Gew.-% weiterer vinylisch copolymerisierbarer Monomere, wie z. B. Butylmethacrylat, Butylacrylat oder Hydroxyethylmethacrylat enthalten sein.

Die genannten Copolymere können in an sich bekannter Weise durch radikalische Substanz-, Lösungs-, Perl- oder Emulsionspolymerisation erhalten werden. Sie müssen vor der Verarbeitung durch geeignete Mahl-, Trocken- oder Sprühprozesse in den erfindungsgemäßen Teilchengrößenbereich gebracht werden.

Dies kann durch einfaches Brechen extudierter und abgekühlter Granulatstränge oder Heißabschlag erfolgen.

Das (Meth)acrylat-Copolymer für den äußeren Überzug c) kann als organische Lösung oder als Dispersion vorliegen und verarbeitet werden.

Bevorzugt setzt man das (Meth)acrylat-Copolymer für den inneren Überzug b) in Form einer Dispersion, mit einem Feststoffgehalt von 10 - 70 Gew.-% ein.

Das (Meth)acrylat-Copolymere c) liegt bevorzugt in Form einer Dispersion z. B. mit einem Wassergehalt von 60 bis 80 Gew.-% vor. Die Carboxylgruppen können bis zu 30-mol%, bevorzugt zu 5 bis 15-mol% durch eine Base, z. B. NaOH, teilneutralisiert sein.

Die Erzeugung der inneren Schicht b) erfolgt bevorzugt durch wäßriges Sprühen einer Wirkstoff-haltigen (Meth)acrylat-Copolymer-Dispersion auf Kerne, z. B. Saccharose Pellets, unter Bindung des Wirkstoffs nach dem Abdampfen bzw. der Verflüchtigung des Wassers. Die Produkttemperatur während des Sprühauftrages kann dabei z. B. 20 bis 40, bevorzugt 25 bis 35 °C betragen. In der Regel ist es nicht notwendig der Wirkstoff-haltigen (Meth)acrylat-Copolymer-Dispersion ein Trennmittel, z. B. Talkum, und einen Weichmacher, z. b. Triethylcitrat, zuzusetzen. Die Verarbeitung des Wirkstoffs kann bevorzugt durch Einrühren in Wasser unter zunächst heftiger Durchmischung, z. B. durch 5 bis 15 minütiges Mischen z. B. mit einem Hochgeschwindigkeitsmixer (Homogenisator) erfolgen. Die so erhaltene Suspension kann dann der (Meth)acrylat-Copolymer-Dispersion zugegeben werden. Die Mischung sollte zweckmäßigerweise und bevorzugt auch während des Sprühvorgangs kontinuierlich gerührt werden.

Die Schichtdicke des inneren Überzugs kann bevorzugt 10 - 300 µm betragen.

### Verschiedene Hilfsstoffe

### Trennmittel

Trennmittel haben folgende Eigenschaften: sie verfügen über große spezifische Oberflächen, sind chemisch inert, sind gut rieselfähig und feinteilig. Aufgrund dieser Eigenschaften lassen sie sich vorteilhaft in Schmelzen homogen verteilen und erniedrigen die Klebrigkeit von Polymeren, die als funktionelle Gruppen stark polare Comonomere enthalten.

### Beispiele für Trockenstellmittel sind:

Aluminiumoxid, Magnesiumoxid, Kaolin, Talkum, Kieselsäure (Aerosile), Bariumsulfat, Ruß und Cellulose.

### Weitere Beispiele für Trennmittel sind:

Ester von Fettsäuren oder Fettsäureamide, aliphatische, langkettige Carbonsäuren, Fettalkohole sowie deren Ester, Montan- oder Paraffinwachse und Metallseifen, insbesondere zu nennen sind Glycerolmonostearat (GMS), Stearylalkohol, Glycerolbehensäureester, Cetylalkohol, Palmitinsäure, Kanaubawachs, Bienenwachs etc..

In der Regel enthält die innere Schicht b) nicht mehr als 1 Gew.-%, bevorzugt kein Trennmittel. Falls ein Trennmittel verwendet wird, ist Glycerolmonostearat bevorzugt.

Weichmacher: Als Weichmacher geeignete Stoffe haben in der Regel ein Molekulargewicht zwischen 100 und 20 000 und enthalten eine oder mehrere hydrophile Gruppen im Molekül, z. B. Hydroxyl-, Ester- oder Aminogruppen. Geeignet sind Citrate, Phthalate, Sebacate, Rizinusöl. Beispiele geeigneter Weichmacher sind Citronensäurealkylester, Propylenglykol, Glycerinester, Phthalsäurealkylester, Sebacinsäurealkylester, Sucroseester, Sorbitanester, Diethylsebacat, Dibutylsebacat und Polyethylenglykole 4000 bis 20.000. Bevorzugte Weichmacher sind Tributylcitrat, Triethylcitrat (TEC), Acetyltriethylcitrat, Dibutylsebacat und Diethylsebacat. Die Einsatzmengen in der äußeren Schicht c) können zwischen 0 und 35, bevorzugt 2 bis 10 Gew.-%, bezogen auf das (Meth)acrylat-Copolymere liegen. Die innere Schicht b) enthält in der Regel höchstens 20 Gew.-%, bevorzugt nicht mehr 12 Gew.-% und besonders bevorzugt keinen Weichmacher.

Weitere pharmazeutisch übliche Hilfsstoffe: Hier sind z. B, Stabilisatoren, Farbstoffe, Antioxidantien, Netzmittel, Pigmente, Glanzmittel etc. zu nennen. Sie dienen vor allem als Verarbeitungshilfsmittel und sollen ein sicheres und reproduzierbares Herstellungsverfahren sowie gute Langzeitlagerstabilität gewährleisten werden kann. Weitere pharmazeutisch übliche Hilfsstoffe können in Mengen von 0,001 Gew-% bis 30 Gew.-%, bevorzugt 0,1 bis 10 Gew.-% bezogen auf das Copolymere vorliegen.

### Freigabeprofile in hypotonem und isotonem Medium

Die erfindungsgemäße mehrschichtige Arzneiform hat insbesondere die Eigenschaft, daß die prozentualen Wirkstofffreigabe-Werte in einem hypotonen und einem isotonen Freigabemedium, basierend auf Phosphatpuffer pH 6,8, im Zeitraum von 1 bis 5 Stunden zu keinem Zeitpunkt um mehr als 10 %, bevorzugt um nicht mehr als 5 % voneinander abweichen. Als hypotones Medium kann Phosphatpuffer pH 6,8 mit einer osmotischen Konzentration von 80 Osmol verwendet werden. Als isotones Medium kann Phosphatpuffer pH 6,8 verwendet werden, bei dem durch Zusatz von NaCl eine osmotische Konzentration von 300 Osmol eingestellt ist.

Die mehrschichtige Arzneiform kann weiterhin, dadurch charakterisiert werden, daß sie im Freisetzungstest nach USP zwei Stunden bei pH 1,2 und anschließendem Umpuffern auf pH 7,0 den enthaltenen Wirkstoff im Zeitraum bis 2,0 Stunden nach Testbeginn zu weniger als 5 % und zum Zeitpunkt acht Stunden nach Testbeginn zu 30 bis 100 % freisetzt.

### BEISPIELE

### Beispiel 1 - 3: Versuchsbeschreibung zu Sprüheinbettung von Budesonid in EUDRAGIT^{®} NE 30 D (Copolymer aus 65 Gew.-% Ethylacrylat und 35 Gew.-% Methylmethacrylat)

Untersucht wurde, ob durch eine Sprüheinbettung eine Freigabeverzögerung erzielt werden kann, die den therapeutischen Anforderungen genügt. Die Formulierungen wurden dazu bezüglich des Wirkstoff-Polymerverhältnisses und der Polymerauftragsmengen variiert. Es wurden im Einzelnen folgende Verhältnisse Polymer zu Budesonid hergestellt: 2,5:1 und 1,6:1.

Alle Formulierungen wurden mit 3% (m/m) Polymerauftrag versehen. Bei Beispiel 1(EUDRAGIT^{®} NE 30 D : Budesonid 2,5:1) und Beispiel 2 (EUDRAGIT^{®} NE 30 D : Budesonid 1,6:1) wurde eine Probe jeweils bei 1% und 2% Polymerauftrag genommen. Alle Chargen wurden nach der Herstellung mit 0,5% Aerosil 200 vermischt, um ein Verkleben der Pellets, während der Lagerung zu verhindern. Vermutlich besitzt der Wirkstoff Budesonid die Wirkung eines Trennmittels. Die Trennwirkung von Budesonid wurde überprüft, in dem in Beispiel 3 (EUDRAGIT^{®} NE 30 D : Budesonid 1,6:1) komplett auf die Verwendung von Talkum als Trennmittel verzichtet wurde.

### Verfahrensbedingungen / Rezepturen:

Budesonidfixierung durch Sprüheinbettung mit EUDRAGIT^{®} NE 30D (Untersuchung der Einflüsse des Verhältnisses Polymer:Budesonid in der Polymerwirkstoffeinbettung). Einwaagen in Gramm.

| Beispiel | 1 | 2 | 3 |
|---|---|---|---|
| Eudragit® NE 30D | 40 | 40 | 13,3 |
| Budesonid | 4,8 | 7,5 | 2,4 |
| Talkum | 6 | 6 | - |
| Wasser | 166,2 | 159 | 55,4 |
| Summe | 217 | 212,5 | 71,1 |
| Polymer:Budesonid Verhältnis | 2,5:1 | 1,6:1 | 1,6:1 |
| Probennahme entsprechend % Polymerauftrag | 1, 2 | 1, 2 | - |
| Lacktrockensubstanz (LTS) [g] | 12 | 12 | 4 |
| Weichmacher bezogen auf LTS | - | - | - |
| Trennmittel bezogen auf LTS | 50% | 50% | - |
| Feststoffgehalt Dispersion (m/m) | 10,5% | 12,0% | 9,0% |
| LTS bezogen auf die Kernmasse | 3% | 3% | 1% |
| Überzugsgerät | Strea | 1 Strea 1 | Strea 1 |
| Düsendurchmesser [mm] | 0,8 | 0,8 | 0,8 |
| Sprühdruck [bar] | 0,5 | 0,5 | 0,5 |
| Ansatzgröße [g] | 400 | 400 | 400 |
| Auftragsmenge [g] | 217 | 212,5 | 71,1 |
| Vorwärmzeit [min] | 5 | 5 | 5 |
| Sprühzeit [min] | 99 | 122 | 50 |
| Zulufttemperatur [°C] | 27 | 27 | 27 |
| Ablufttemperatur [°C] | 23 | 23 | 23 |
| Sprührate [g/min] | 2,19 | 1,74 | 1,42 |
| Nachtrockenzeit [min] | 5 | 5 | 5 |

### Beispiel 4 (Überzug zu Steuerung der Freigabe im Kolon)

Verfahrensbedingungen/Rezepturen:Überzugsversuche mit EUDRAGIT^{®} FS 30D (Copolymer aus 65 Gew.-% Methylacrylat, 25 Gew.-% Methylmethacrylat und 10 Gew.-% Methacrylsäure) auf retardierende Budesonidpellets mit NE Wirkstoffeinbettung. (Einwaagen in Gramm).

| Beispiel | 4 |
|---|---|
| Ausgangscharge | Versuch 1 |
| EUDRAGIT^{®} FS 30D | 233,3 |
| GMS | 3,5 |
| TEC | 3,5 |
| Tween 80 | 1,4 |
| Wasser | 191,0 |
| Summe | 435,56 |
| Probe bei % LTS | 10; 15 |
| Lacktrockensubstanz (LTS) [g] | 70 |
| Weichmacher bezogen auf LTS [%] | 5,0 |
| Trennmittel bezogen auf LTS | 5,0% |
| Feststoffgehalt Dispersion (m/m) [%] | 18,0 |
| LTS bezogen auf die Kernmasse | 20% |
| Überzugsgerät | Strea 1 |
| Düsendurchmesser [mm] | 0,8 |
| Sprühdruck [bar] | 0,5 |
| Ansatzgröße [g] | 350 |
| Auftragsmenge [g] | 435,6 |
| Vorwärmzeit [min] | 5 |
| Sprühzeit [min] | 150 |
| Zulufttemperatur [°C] | 41 |
| Ablufttemperatur [°C] | 30 |
| Sprührate [g/min] | 3,0 |
| Nachtrockenzeit [min] | 5 |

### Beispiel 5: (Überzug zur Steuerung der Freigabe im Intestinum)

### Verfahrensbedingungen/Rezeptur:

Magensaftresistenter Überzug mit Eudragit® L 30 D-55 (Copolymer aus 50 Gew.-% Ethylacrylat und 50 Gew.-% Methacrylsäure) auf retardierte Budesonidpellets. Einwaagen erfolgten in Gramm.

| Beispiel | 5 |
|---|---|
| Ausgangscharge | Versuch 3 |
| EUDRAGIT^{®} L 30D-55 | 233 |
| Talkum | 35 |
| TEC | 7 |
| Wasser | 285 |
| Summe | 560 |
| Probe bei % LTS | 10%, 15% |
| Lacktrockensubstanz (LTS) [g] | 70 |
| Weichmacher bezogen auf LTS | 10% |
| Trennmittel bezogen auf LTS | 50% |
| Feststoffgehalt Dispersion (m/m) | 20,0% |
| LTS bezogen auf die Kernmasse | 20% |
| Überzugsgerät | Strea 1 |
| Düsendurchmesser [mm] | 0,8 |
| Sprühdruck [bar] | 0,5 |
| Ansatzgröße [g] | 350 |
| Auftragsmenge [g] | 560 |
| Vorwärmzeit [min] | 5 |
| Sprühzeit [min] | 248 |
| Zulufttemperatur [°C] | 44 |
| Ablufttemperatur [°C] | 31 |
| Sprührate [g/min] | 2,56 |
| Nachtrockenzeit [min] | 5 |

### Ergebnis der Untersuchung

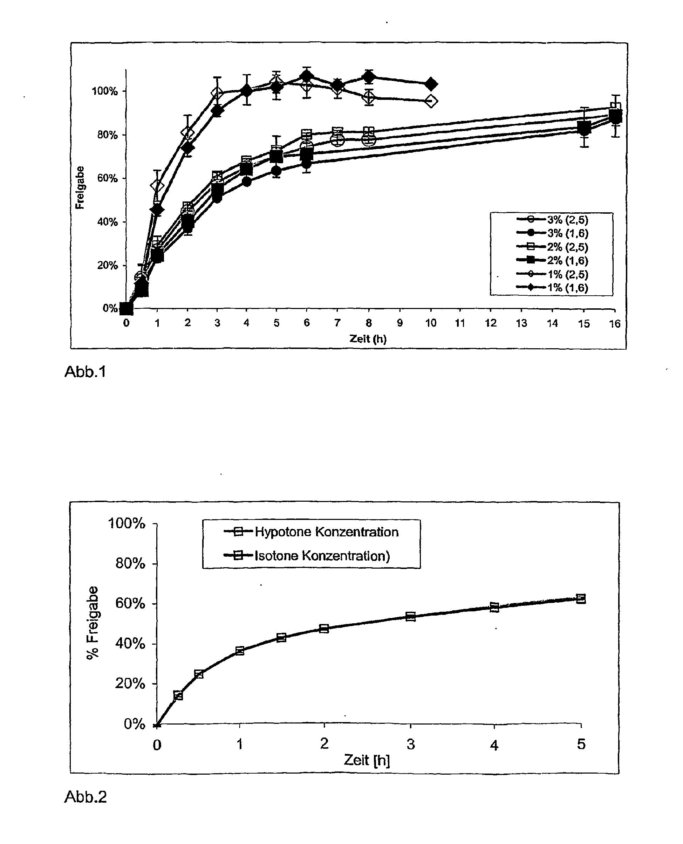

In Abb. 1 sind die vergleichenden Freigabeprofile des Beispiels 1 (EUDRAGIT^{®} NE 30 D : Budesonid 2,5:1) und Beispiels 2 (EUDRAGIT^{®} NE 30 D : Budesonid 1,6:1) in Phosphatpuffer pH 6,8 dargestellt. Die Freigaberate nimmt mit steigender Polymerauftragsmenge, gleichbedeutend mit zunehmender Filmdicke, ab. Die Freigabe für die 1%igen Polymeraufträge verläuft innerhalb von 3-4 Stunden quantitativ. Bei größeren LTS Auftrag kann eine Erniedrigung der Freigaberate beobachtet werden.
Nach einer beschleunigten Freigaberate zu Beginn der Freisetzung gehen die Profile in eine lineare Freigabekinetik mit niedrigerer Freigabegeschwindigkeit über. Die Beispiele 1 und 2 mit 2% respektive 3% (m/m) Polymerauftrag setzten nach 16 Stunden zwischen 87,2% und 92,5% der Dosis frei.

Bei einer sehr niedrigen Auftragsmenge (1% m/m Lacktrockensubstanz (LTS)) ist noch kein homogen geschlossener Film zu erwarten. Der Wirkstoff liegt vielmehr polymerfixiert, in einem sehr "lockeren" Netzwerk auf der Oberfläche der non pareils vor. Der an der Oberfläche der Matrix präsentierte Wirkstoff, steht mit dem Auflösungsmedium in direktem Kontakt. Suspendiertes Budesonid in der Matrix, muss dagegen nach der Auflösung erst durch die Polymerstruktur diffundieren, um, dem Konzentrationsgradienten folgend, in das umgebende Freigabemedium zu gelangen. Da das Verhältnis der Oberfläche zur Polymermatrix bei einem niedrigeren Polymerauftrag höher ist, kann damit die erhöhte initiale Freigabe erklärt werden. Ein größerer Anteil der Wirkstoffdosis befindet sich an der Oberfläche der Matrix und wird im Verhältnis schnell freigesetzt. Die verlangsamte Freigabe bei 3% gegenüber 2% könnte damit erklärt werden, dass die Masse der Polymermatrix, und in folge dessen, auch die Dicke der Matrix, signifikant bei einer Erhöhung des Polymerauftrags zunimmt, während die Oberfläche kaum beeinflusst wird. Der mittlere Diffusionsweg verlängert sich und die Freigabe wird folglich verlangsamt.

**Abb.1** Freigabeprofile zweier Chargen unterschiedlichem Polymer:Wirkstoff-Verhältnis (2,5:1, Versuch 1) und (1,6:1, Versuch 2) mit unterschiedlichen Polymerauftragsmengen in Phosphatpuffer pH 6,8. Der Quotient des jeweiligen Polymer:Wirkstoff-Verhältnisses ist in der Legende in runden Klammen gesetzt.

### Robustheit des Freigabeverhaltens

3%ige Polymerüberzüge des Beispiels 1 (Eudragit NE 30 D : Budesonid 2,5:1) wurden auf ihre Robustheit hinsichtlich der osmotischen Konzentration des Freigabemediums untersucht. Als Dissolution-Medium wurde Phosphatpuffer pH 6,8, mit einer osmotischen Konzentration von 80 mOsmol und 300 mOsmol verwendet. Eine annähernd isotone Konzentration von 300 mOsmol wurde durch Zusatz von NaCl zum Puffer eingestellt. Dieser Osmolaritätsbereich deckt die präprandialen Zustände im proximalen Gl-Trakt mit und ohne gleichzeitige Einnahme der Pellets mit bis zu 250 ml Wasser ab. Es ist zu beobachten, dass die Osmolarität keinen Einfluss auf die Freigabe aus den Pellets hat. Die Freigabe verläuft sehr robust (Abb. 2).

**Abb. 2****:** Freigabeprofile von Beispiel 1 mit 3% (m/m) Polymerauftrag in Phosphatpuffer und einer isotonen und hypotonen Osmolarität.

### Beispiel 4: Modifikation des Beginns der Freigabe durch ein Filmcoating mit Eudragit® FS 30 D

Die Überzugscharge entspricht Beispiel 1 (Sprüheinbettung von Budesonid in Eudragit NE 30 D, Polymer:Wirkstoff Verhältnis 2,5:1, 3% m/m LTS) wurde mit Eudragit FS 30 D zur Modifikation des Beginns der Freigabe überzogen. Die resultierende Charge (2-24)wurde genauer, hinsichtlich ihres *in vitro* Freigabeverhaltens, untersucht. Ziel war die verlangsamte Freigabe von Budesonid, wobei die Freisetzung erst im terminalen Dünndarm einsetzen soll.

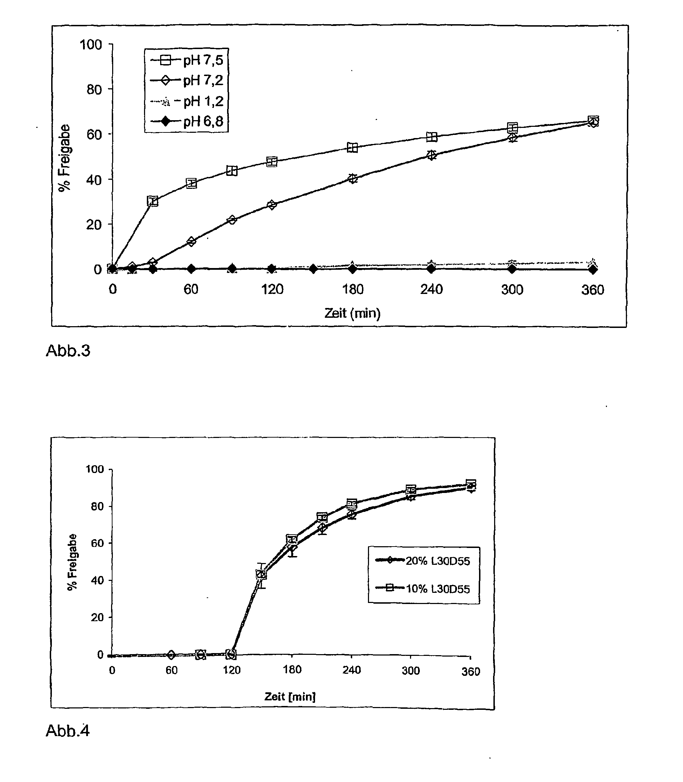

Die durchgeführten Freigabeuntersuchungen in Arzneibuchpuffern mit pH 1,2; 6,8; 7,2 und 7,5 zeigen für Versuch 4 mit 20% (m/m) LTS von Eudragit® FS 30 D eine Unterbindung der Freigabe bei pH 1,2 und 6,8. Bei pH-Werten also, die den Magen und den proximalen Dünndarm simulieren sollen. Die Freisetzung setzt, mit einer kurzen t_{lag} Phase zwischen 15 bis 30 Minuten, in Puffer pH 7,2 ein. Die Freigabe folgt danach einem retardierten nahezu linearen Kurvenverlauf. Das äußere Polymer löst sich in bei diesem pH noch nicht auf, jedoch ist die Quellung stark ausgeprägt. Die Freigabe wird in diesem Fall von der Diffusion durch das gequollene Polymer kontrolliert. Bei pH 7,5 beginnt die Freigabe sofort, ohne, dass eine lag time beobachtet werden kann. Das äußere Polymer Eudragit® FS 30 D löst sich rasch auf und die Freigabe wird allein durch die Wirkstoffeinbettung in Eudragit® NE 30 D gesteuert (Abb. 3)

**Abbildung 3****.** Freigabeprofile von Versuch 4 (20% (m/m) Eudragit® FS 30 D Überzug auf Budesonidsprüheinbettung in Eudragit® NE 30 D (Polymer:Wirkstoffverhältnis 2,5:1)) in Arzneibuchpuffern mit unterschiedlichen pH-Werten.

### Beispiel 5: Magensaftresistenter Überzug mit Eudragit® L 30 D-55

Versuch 5 wurde als Prototyp für die Therapie des Morbus Crohn ausgewählt und durch *in vitro* Freigabeuntersuchungen genauer charakterisiert. Die Charge setzt sich aus einer Sprüheinbettung des Budesonid in Eudragit® NE 30 D mit 1% (m/m) LTS Auftrag und einem magensaftresistenten Überzugspolymer, nämlich Eudragit® L 30 D-55 mit 10%, respektive 20% LTS-Auftrag zusammen. Da der magensaftresistente Polymerüberzug in direktem Kontakt zur Einbettungsmatrix steht, war es von Interesse, einen möglichen Einfluss des Überzugs auf die Freisetzung aus der Einbettung zu untersuchen. Die Prüfung auf Magensaftresistenz wurde nach der Monographie des USP 24 "Delayedrelease (Enteric-coated) Articles - General Drug Release Standard", Methode A durchgeführt. Es konnte weder bei 10% noch 20% (m/m) Polymerauftrag eine Freigabe über 2 Stunden in simuliertem Magensaft gemessen werden. Nach Pufferung auf pH 6,8 setzt die Freisetzung ohne Verzögerung ein. Es ist zu beobachten, dass das Freigabeprofil durch den höheren, 20%igen Polymerauftrag kaum gegenüber dem 10%igen LTS-Auftrag beeinflusst wird.

**Abbildung 4****.** Freigabeprofil von Beispiel 5 mit 10% und 20% (m/m) Polymerauftrag EUDRAGIT^{®} L 30 D-55. Freigabe für 2 Stunden in 0,1 N HCl und danach Umpufferung auf pH 6,8.

**Abbildung 5****:** Freigabeprofile von Versuch 5 in Dissolution-Medien (Phosphatpuffer, Ph 6,8) mit unterschiedlicher Osmolarität. Einbettung in Eudragit® NE 30 D (Versuch 3) und Coating mit Eudragit® L 30 D (Copolymer aus 50 Gew.-% Methylmethacrylat und 50 Gew.-% Methacrylsäure)

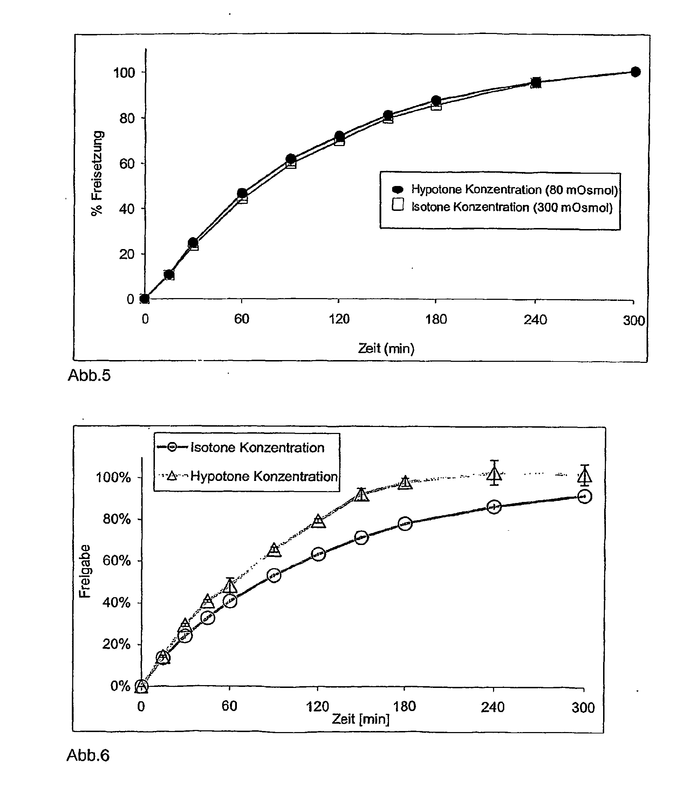

Die Freigabeuntersuchung, deren Ergebnisse graphisch in Abb. 5 dargestellt sind, zeigt, dass sich die Freigabe aus den Pellets sehr robust gegenüber Veränderungen des Freigabemediums verhält. Ein osmotischer Einfluss auf das Freisetzungsprofil im Bereich von 80 bis 300 mOsmol kann in den Versuchen praktisch nicht beobachtet werden.

### Beispiel 6 (nicht erfindungsgemäß, Vergleich zu Beispiel 1)

Formulierung Vergleich zu Beispiel 1.) mit Eudragit RL 30D als retardierender Überzug und EUDRAGIT^{®} L 30 D-55 als magensaftresistenter Filmüberzug (MR). Einwaagen in Gramm.

| Beispiel 6 | Retard | MR |
|---|---|---|
| Eudragit RL 30D | 200 | ---- |
| Eudragit L 30D-55 | ---- | 167 |
| Talkum | 30 | 25 |
| TEC | 12 | 5 |
| Wasser | 268 | 203 |
| Gesamt | 510 | 400 |
| Lacktrockensubstanz (LTS) [g] | 60 | 50 |
| Weichmacher bezogen auf LTS | 20% | 10% |
| Trennmittel bezogen auf LTS | 50% | 50% |
| Feststoffgehalt Dispersion (m/m) | 20,4% | 20% |
| LTS bezogen auf die Kernmasse | 12% | 20% |
| Überzugsgerät | Strea 1 | Strea 1 |
| Düsendurchm. [mm] | 0,8 | 0,8 |
| Sprühdruck [bar] | 0,5 | 0,5 |
| Ansatzgröße [g] | 500 | 250 |
| Auftragsmenge [g] | 510 | 400 |
| Vorwärmzeit [min] | 5 | 5 |
| Sprühzeit [min] | 213 | 167 |
| Zulufttemperatur [°C] | 34 | 40 |
| Ablufttemperatur [°C] | 25 | 30 |
| Sprührate [g/min] | 2,4 | 2,4 |
| Nachtrockenzeit [min] | 5 | 10 |

**Abbildung 6****:** Freigabeprofile von Beispiel 6 Aminosalicylsäurepellets mit einen Retardüberzug EUDRAGIT RL 30 D und einem magensaftresistenten Überzug von EUDRAGIT L 30 D-55 in Phosphatpuffer mit unterschiedlicher Osmolarität.

Alle Freigabetests wurden nach USP Methode 2 (Paddle) mit einer Drehzahl von 100 Upm durchgeführt.

## Patentansprüche

1. Mehrschichtige Arzneiform, aufgebaut aus
a) einem neutralen Kern,
b) einem inneren Überzug aus einem Methacrylat-Copolymeren
c) einem äußeren Überzug aus einem Copolymeren, das sich aus 40 bis 95 Gew.-% radikalisch polymerisierten C₁- bis C₄-Alkylestern der Acryl- oder der Methacrylsäure und 5 bis 60 Gew.-% (Meth)acrylat-Monomeren mit einer anionischen Gruppe im Alkylrest zusammensetzt
**dadurch gekennzeichnet, daß**
der innere Überzug im wesentlichen aus einem Methacrylat-Copolymeren besteht, das mindestens zu 90 Gew.-% aus (Meth)acrylat-Monomeren mit neutralen Resten aufgebaut ist, eine Mindestfilmbildetemperatur nach DIN 53 787 von höchstens 30 °C aufweist und den pharmazeutischen Wirkstoff in gebundener Form enthält.

2. Mehrschichtige Arzneiform nach Anspruch 1, **dadurch gekennzeichnet, daß** das Methacrylat-Copolymer des inneren Überzugs aus 25 - 35 Gew.-% Methylmethacrylat, 75 bis 65 Gew.-% Ethylacrylat und gegebenenfalls bis zu 10 Gew.-% anderen vinylisch polymerisierbare Monomeren, insbesondere (Meth)acrylatmonomeren mit polaren oder ionischen Resten polymerisiert ist, wobei sich die Mengenanteile zu 100 Gew.-% addieren.

3. Mehrschichtige Arzneiform nach Anspruch 1 oder 2, **dadurch gekennzeichnet, daß** das Wirkstoff/Polymerverhältnis der inneren Schicht 20 zu 1 bis 1 zu 20 beträgt.

4. Mehrschichtige Arzneiform nach einem oder mehreren der Ansprüche 1 bis 3, **dadurch gekennzeichnet, daß** der äußere Überzug im wesentlichen aus einem (Meth)acrylat Copolymeren aus 40 bis 60, Gew.-% Methacrylsäure und 60 bis 40 Gew.-% Methylmethacrylat oder 60 bis 40 Gew.-% Ethylacrylat besteht.

5. Mehrschichtige Arzneiform nach einem oder mehreren der Ansprüche 1 bis 3, **dadurch gekennzeichnet, daß** der äußere Überzug im wesentlichen aus einem (Meth)acrylat Copolymeren aus 20 bis 40 Gew.-% Methacrylsäure und 80 bis 60 Gew.-% Methylmethacrylat besteht.

6. Mehrschichtige Arzneiform nach einem oder mehreren der Ansprüche 1 bis 3, **dadurch gekennzeichnet, daß** der äußere Überzug im wesentlichen aus einem (Meth)acrylat Copolymeren aus 20 bis 34 Gew.-% Methacrylsäure und/oder Acrylsäure, 20 bis 69 Gew.-% Methylacrylat, 0 bis 40 Gew.-% Ethylacrylat und gegebenenfalls 0 bis 10 Gew.-% weiteren vinylisch copolymerisierbarern Monomeren, mit der Maßgabe, daß die Glastemperatur des Copolymers nach ISO 11357-2, Punkt 3.3.3, höchstens 60 °C beträgt, besteht.

7. Mehrschichtige Arzneiform nach einem oder mehreren der Ansprüche 1 bis 3 **dadurch gekennzeichnet, daß** der äußere Überzug im wesentlichen aus einem (Meth)acrylat Copolymeren, bestehend aus 10 bis 30 Gew.-%, Methylmethacrylat, 50 bis 70 Gew.-% Methylacrylat und 5 bis 15 Gew.-% Methacrylsäure besteht.

8. Mehrschichtige Arzneiform nach einem oder mehreren der Ansprüche 1 bis 7, **dadurch gekennzeichnet, daß** sie einen Wirkstoff aus den Wirkstoffklassen der Aminosalicylate, der Sulfonamide oder der Glucocorticoide enthält.

9. Mehrschichtige Arzneiform nach Anspruch 8, **dadurch gekennzeichnet, daß** sie den Wirkstoff 5-Aminosalicylsäure, Olsalazin, Sulfalazin, Prednison, Prednisolon oder Budesonid enthält.

10. Mehrschichtige Arzneiform nach einem oder mehreren der Ansprüche 1 bis 7, **dadurch gekennzeichnet, daß** sie einen Wirkstoff aus den Wirkstoffklassen der Enzyme, Peptidhormone, immunmodulatorische Proteine, Antigene, Antikörper oder der Oligonukleotide enthält.

11. Mehrschichtige Arzneiform nach Anspruch 10, **dadurch gekennzeichnet, daß** sie den Wirkstoff Pankreatin, Insulin, Human Growth Hormon (hGH), Corbaplatin, Intron A, Calcitonin, Cromalyn, Interferone, Calcitonin, Granulocyte Colony Stimulating factor (G-CSF), Interleukin, Parathyroidhormone, Glucagon, Pro-Somatostatin, Somatostatin, Detirelix, Cetrorelix, Vasopressin, 1-Deaminocysteine-8-D-arginine-Vasopressin, Leuprolidacetat oder ein Antigen, das aus Gräsern oder anderen Pflanzen, wie z. B. Roggen, Weizen, Gerste, Hafer, Bermuda Gras, Zinnkraut, Ahorn, Ulme, Eiche, Platane, Pappel, Zeder, Zinnkraut, Disteln gewonnen wurde, enthält.

12. Mehrschichtige Arzneiform nach einem oder mehreren der Ansprüche 1 bis 6, **dadurch gekennzeichnet, daß** die prozentualen Wirkstofffreigabe-Werte in einem hypotonen und einem isotonen Freigabemedium basierend auf Phosphatpuffer pH 6,8 im Zeitraum von 1 bis 5 Stunden zu keinem Zeitpunkt um mehr als 10 % voneinander abweichen.

## Claims

1. Multilayer dosage form composed of
a) a neutral core,
b) an inner coating of a methacrylate copolymer
c) an outer coating of a copolymer which is composed of 40 to 95% by weight free-radical polymerized C₁- to C₄-alkyl esters of acrylic or of methacrylic acid and 5 to 60% by weight (meth)acrylate monomers having an anionic group in the alkyl radical,
**characterized in that**
the inner coating consists substantially of a methacrylate copolymer which is composed of at least 90% by weight of (meth)acrylate monomers having neutral radicals, has a minimum film-forming temperature as specified in DIN 53 787 not exceeding 30°C, and comprises the pharmaceutical active substance in bound form.

2. Multilayer dosage form according to Claim 1, **characterized in that** the methacrylate copolymer of the inner coating is polymerized from 25-35% by weight methyl methacrylate, 75 to 65% by weight ethyl acrylate and, where appropriate, up to 10% by weight other vinylically polymerizable monomers, in particular (meth)acrylate monomers with polar or ionic radicals, where the proportionate amounts add up to 100% by weight.

3. Multilayer dosage form according to Claim 1 or 2, **characterized in that** the active substance/polymer ratio of the inner layer is from 20:1 to 1:20.

4. Multilayer dosage form according to one or more of Claims 1 to 3, **characterized in that** the outer coating consists substantially of a (meth)acrylate copolymer of 40 to 60% by weight methacrylic acid and 60 to 40% by weight methyl methacrylate or 60 to 40% by weight ethyl acrylate.

5. Multilayer dosage form according to one or more of Claims 1 to 3, **characterized in that** the outer coating consists substantially of a (meth)acrylate copolymer of 20 to 40% by weight methacrylic acid and 80 to 60% by weight methyl methacrylate.

6. Multilayer dosage form according to one or more of Claims 1 to 3, **characterized in that** the outer coating consists substantially of a (meth)acrylate copolymer of 20 to 34% by weight methacrylic acid and/or acrylic acid, 20 to 69% by weight methyl acrylate, 0 to 40% by weight ethyl acrylate and, where appropriate, 0 to 10% by weight further vinylically copolymerizable monomers, with the proviso that the glass transition temperature of the copolymer as specified in ISO 11357-2, subsection 3.3.3, does not exceed 60°C.

7. Multilayer dosage form according to one or more of Claims 1 to 3, **characterized in that** the outer coating consists substantially of a (meth)acrylate copolymer consisting of 10 to 30% by weight methyl methacrylate, 50 to 70% by weight methyl acrylate and 5 to 15% by weight methacrylic acid.

8. Multilayer dosage form according to one or more of Claims 1 to 7, **characterized in that** it comprises an active substance from the active substance classes of aminosalicylates, of sulfonamides or of glucocorticoids.

9. Multilayer dosage form according to Claim 8, **characterized in that** it comprises the active substance 5-aminosalicylic acid, olsalazine, sulfasalazine, prednisone, prednisolone or budesonide.

10. Multilayer dosage form according to one or more of Claims 1 to 7, **characterized in that** it comprises an active substance from the active substance classes of enzymes, peptide hormones, immunomodulatory proteins, antigens, antibodies or of oligonucleotides.

11. Multilayer dosage form according to Claim 10, **characterized in that** it comprises the active substance pancreatin, insulin, human growth hormone (hGH), carboplatin, intron A, calcitonin, cromalyn, interferons, granulocyte colony stimulating factor (G-CSF), interleukin, parathyroid hormones, glucagon, pro-somatostatin, somatostatin, detirelix, cetrorelix, vasopressin, 1-deaminocysteine-8-D-arginine-vasopressin, leuprolide acetate or an antigen which has been isolated from grasses or other plants such as, for example, rye, wheat, barley, oats, bermuda grass, horsetail, sycamore, elm, oak, plane tree, poplar, cedar, thistles.

12. Multilayer dosage form according to one or more of Claims 1 to 6, **characterized in that** the values for the percentage release of active substance in a hypotonic and an isotonic release medium based on phosphate buffer pH 6.8 do not differ from one another at any time in the period from 1 to 5 hours by more than 10%.

## Revendications

1. Forme médicamenteuse à plusieurs couches, formée à partir de
a) un noyau neutre,
b) un revêtement interne en un copolymère de méthacrylate
c) un revêtement externe en un copolymère qui est composé par 40 à 95% en poids d'esters C₁-C₄-alkyliques de l'acide acrylique ou méthacrylique polymérisés par voie radicalaire et 5 à 60% en poids de monomères de type (méth)acrylate avec un groupe anionique dans le radical alkyle
**caractérisée en que** le revêtement interne est essentiellement constitué par un copolymère de méthacrylate, qui est formé, à raison d'au moins 90%, à partir de monomères de type (méth)acrylate avec des radicaux neutres, présente une température minimale de formation de film selon la norme DIN 53 787 d'au plus 30°C et contient la substance active pharmaceutique sous forme liée.

2. Forme médicamenteuse à plusieurs couches selon la revendication 1, **caractérisée en ce que** le copolymère de méthacrylate du revêtement interne est polymérisé à partir de 25-35% en poids de méthacrylate de méthyle, 75 à 65% en poids d'acrylate d'éthyle et le cas échéant jusqu'à 10% en poids d'autres monomères vinyliquement polymérisables, en particulier des monomères de type (méth)acrylate avec des radicaux polaires ou ioniques, les proportions de quantités s'ajoutant à 100% en poids.

3. Forme médicamenteuse à plusieurs couches selon la revendication 1 à 2, **caractérisée en ce que** le rapport substance active/polymère de la couche interne est de 20:1 à 1:20.

4. Forme médicamenteuse à plusieurs couches selon l'une ou plusieurs des revendications 1 à 3, **caractérisée en ce que** le revêtement externe est constitué essentiellement par un copolymère de (méth)acrylate formé à partir de 40 à 60% en poids d'acide méthacrylique et 60 à 40% en poids de méthacrylate de méthyle ou 60 à 40% en poids d'acrylate d'éthyle.

5. Forme médicamenteuse à plusieurs couches selon l'une ou plusieurs des revendications 1 à 3, **caractérisée en ce que** le revêtement externe est constitué essentiellement par un copolymère de (méth)acrylate formé à partir de 20 à 40% en poids d'acide méthacrylique et 80 à 60% en poids de méthacrylate de méthyle.

6. Forme médicamenteuse à plusieurs couches selon l'une ou plusieurs des revendications 1 à 3, **caractérisée en ce que** le revêtement externe est essentiellement constitué par un copolymère de (méth)acrylate formé à partir de 20 à 34% en poids d'acide méthacrylique et/ou d'acide acrylique, 20 à 69% en poids d'acrylate de méthyle, 0 à 40% en poids d'acrylate d'éthyle et le cas échéant 0 à 10% en poids d'autres monomères vinyliquement copolymérisables, à condition que la température de transition vitreuse du copolymère selon la norme ISO 11357-2, article 3.3.3, soit d'au plus 60°C.

7. Forme médicamenteuse à plusieurs couches selon l'une ou plusieurs des revendications 1 à 3, **caractérisée en ce que** le revêtement externe est essentiellement constitué par un copolymère de (méth)acrylate constitué par 10 à 30% en poids de méthacrylate de méthyle, 50 à 70% en poids d'acrylate de méthyle et 5 à 15% en poids d'acide acrylique.

8. Forme médicamenteuse à plusieurs couches selon l'une ou plusieurs des revendications 1 à 7, **caractérisée en ce qu'**elle contient une substance active de la classe de substances actives des aminosalicylates, des sulfonamides ou des glucocorticoïdes.

9. Forme médicamenteuse à plusieurs couches selon la revendication 8, **caractérisée en ce qu'**elle contient la substance active pharmaceutique acide 5-aminosalicylique, olsalazine, sulfasalazine, prednisone, prednisolone ou budésonide.

10. Forme médicamenteuse à plusieurs couches selon l'une ou plusieurs des revendications 1 à 7, **caractérisée en ce qu'**elle contient une substance active des classes de substances actives des enzymes, des hormones peptidiques, des protéines d'immunomodulation, des antigènes, des anticorps ou des oligonucléotides.

11. Forme médicamenteuse à plusieurs couches selon la revendication 10, **caractérisée en ce qu'**elle contient, comme substance active, la pancréatine, l'insuline, l'hormone de croissance humaine (hGH), le carboplatine, l'Intron A, la calcitonine, la cromolyne, un interféron, un facteur de stimulation des colonies de granulocytes (G-CSF), une interleukine, une hormone parathyroïdienne, le glucagon, la pro-somatostatine, une somatostatine, le detirelix, le cetrorelix, la vasopressine, la 1-désaminocystéine-8-D-arginine-vasopressine, l'acétate de leuprolide ou un antigène, qui est produit à partir de graminées ou d'autres plantes, telles que par exemple le seigle, le blé, l'orge, l'avoine, le cynodon dactyle (chiendent cultivé), la prèle, l'érable, l'orme, le chêne, le platane, le peuplier, le cèdre, les chardons.

12. Forme médicamenteuse à plusieurs couches selon l'une ou plusieurs des revendications 1 à 6, **caractérisée**
**en ce que** les valeurs de libération de la substance active en pour cent dans un milieu de libération hypotonique et isotonique à base d'un tampon phosphate de pH 6,8 en un laps de temps de 1 à 5 heures ne diffèrent en aucun moment de plus de 10% les unes des autres.
